# EUROPEAN PATENT APPLICATION

(11) **EP 3 321 251 A2**
(43) Date of publication of application: **16.05.2018**
(21) Application number: 16823920.0
(22) Date of filing: 08.09.2016
(51) Int. Cl.: C07C 273/04, C05C 9/00

(54) **METHOD FOR PRODUCING ORGANIC UREA AND THE ORGANIC UREA AND AUS32 THEREBY OBTAINED**

(30) Priority: 08.07.2015 ES 201530983
(71) Applicant: Estefano Lagarrigue, Roberto, 08480 Ametlla del Vallès (ES)
(72) Inventor: Estefano Lagarrigue, Roberto, 08480 Ametlla del Vallès (ES)
(74) Representative: Espiell Volart, Eduardo Maria
(86) International application number: PCT/ES2016/000093
(87) International publication number: WO 2017/009498

(57) **Abstract**

Method for producing organic urea that, by mixing and reacting liquid ammonia (NH₃) and gaseous carbon dioxide (CO₂) in steps of ammonium carbamate formation (5), carbamate decomposition (7), urea synthesis (8) and evaporation (9) thereof, is carried out using organic ammonia from animal and plant biomass (2) and carbon dioxide recycled from the exhaust gases (6) from combined-cycle biogas boilers with plant biomass, comprising prior steps of methanization (1), with a biomass "digester", mixing with pig and chicken slurry and blood, pine needles and ash, and water, until obtaining: methane gas, air, gaseous ammonia and water vapor and PH regulator and catalyzing (3), where the obtained gases are condensed by cooling and the gaseous ammonia is separated to be stored at a pressure of 13 atmospheres. The invention also claims the organic urea and the chemical reactor AUS32 *(AdBlue*®*)* manufactured using from biomass.

## Description

### OBJECT OF THE INVENTION

The invention relates to a method for producing organic urea as well as organic urea and AUS 32 obtained by this method, as described in the title of the present specification.

The object of the present invention focuses specifically on a method for producing organic urea that is innovatively carried out using biomass as a raw material, specifically using ammonia (NH₃) from biomass of plant and animal origin, by mixing it with the carbon dioxide (CO₂) recycled from the exhaust gases released from a combined-cycle biogas boiler (gas - solids) with plant biomass, in order to form an intermediate compound, which is the one that will produce the organic urea.

### FIELD OF APPLICATION OF THE INVENTION

The field of application of the present invention falls within the sector of the industry dedicated to manufacturing chemical products, fertilizers and domestic and industrial effluent treatment plants, particularly focused on the area of production of organic urea, compost, irrigation water, reducing effluents and gases that produce the greenhouse effect.

### BACKGROUND OF THE INVENTION

As is known, urea is a crystalline and colorless chemical compound, with the formula CO(NH₂)₂. It is found abundantly in urine and fecal matter. It is the main end product of protein metabolism in humans and other mammals. Human urine contains about 20 g per liter. In small amounts, it is present in blood, the liver and lymph and in serous fluids and also in excrement from fish and many other animals. It is also found in the heart, lungs, bones, reproductive organs (semen), fungi, legumes and grains.

Urea is currently useful as a raw material for forming livestock feed, agricultural and livestock fertilizers, moisturizing creams and as AUS 32 (*AdBlue* ®) which is a chemical reactor for the degradation of nitrous oxide in exhaust gases basically generated by internal combustion engines. Obtaining said compound at an industrial level will be conditioned by the formation of carbamates in any of the raw materials thereof (Biomasses and/or Hydrocarbon gas)

The use of urea and phosphate fertilizers enables the crops to become stronger and be able to endure, with the help of the necessary agrochemical products, different types of insects, bacteria and viruses that can affect them during the time it takes for their fruit to mature.

The use of urea inside systems that operate with SCR (Selective Catalytic Reduction) as a main component in the AUS 32 (*AdBlue* ®) chemical reactor (urea and distilled water) enables a significant reduction in the emissions generated by the exhaust gases from internal combustion engines, both mobile and stationary.

Urea, also referred to as carbamide, carbonyldiamide or arbamidic acid, is the name of the carbonic acid of the diamide, the chemical formula of which is (NH₂)2CO.

Moreover, urea synthesis is known at an industrial level (hydrocarbon based) and is made by cracking gas in order to obtain liquid ammonia (NH₃) and gaseous carbon dioxide (CO₂). When said components are mixed, an intermediate product called ammonium carbamate is formed, which, in turn, is dehydrated in order to form urea.

Specifically, the complete process of "traditional production" of urea at an industrial level, in other words, the one applied until now, can be carried out in the following steps:
- Obtaining CO₂.
- Obtaining ammonia
- Carbamate formation
- Degradation of the carbamate and recycling
- Urea synthesis
- Dehydration, concentration and granulation.
- Obtaining CO₂. The CO₂ is obtained from natural gas, by means of the reaction known as reforming.

Before reforming, the impurities must be separated from the gas, such as oil droplets, dust particles and especially desulphurizing the gas, since sulphur interferes with the effects of the catalysts.

After purifying the gas, the CO₂ is then obtained by means of two catalytic reforming steps with water vapor. The heat necessary for the reaction, which is endothermic, comes from the combustion of the natural gas and the partially reformed gases. Air is allowed to enter into the reactor in order to obtain the necessary H₂/N₂ ratio in order to subsequently obtain ammonia. The reaction is as follows:

2 CH₄ + 3 H₂O CO + CO₂ + 7 H₂

The two reforming steps are verified according to the reaction displayed, and at the end of the second step, a gas is obtained with the following proportions: 56% H₂, 12% CO, 8% CO₂, 23% N₂ and less than 0.5% CH₄.

In order to eliminate the CO and convert it into CO₂, the CO conversion is carried out by making it react catalytically with water vapor in order to form CO₂ and H₂, using iron and copper as catalysts.

The CO₂ is separated from the resulting gas by means of a monoethanolamine (MEA) by means of the following reaction:

MEA (CO₂) MEA + CO₂

Compression of the carbon dioxide. The resulting dioxide is sent to two successive compression steps in which the pressure is raised to 160 absolute atmospheres. Small amounts of air are passively added to the dioxide in order to inhibit the corrosive action.
- Obtaining ammonia. After the methanization, the circulating gas is made up of air, methane and water vapor, which react with an iron catalyst in order to form ammonia in a gaseous state according to:

   7 CH₄ + 10 H₂O + 8 N₂ + 2 O₂ 16 NH₃ + 7 CO₂

The gaseous ammonia is condensed by cooling and is separated from the gas to be stored at a pressure of 13 atmospheres. The remaining gaseous ammonia is recirculated to the synthesis loop.
- Urea synthesis. - The reaction is performed at high pressures (200 bar) and the optimal temperature level (190aC) in a reactor constructed from special stainless steel. The reaction is produced between the ammonia, the CO₂ and the recycled carbamate solution, coming from the absorption step. The ammonium carbamate is formed from CO₂ and NH₃ according to the following reaction (this reaction generates heat):

   2NH₃(g) + CO₂(g) NH2 - COONH₄(l) DH= -117 kJ/mol

   Ammonia + Carbonic Gas Ammonium Carbamate

Before entering the reactor, the CO₂ is compressed to 200 atm, by means of an electric compressor, and the ammonia is compressed to 145 atm.

The NH₃ and the CO₂ react quickly and exothermically, in a first step, in order to form the carbamate which is later dehydrated to urea + water. This reaction reaches close to 100% in normal conditions. - Granulation. Then the urea is prilled (formation of small beads about 2-4 mm in diameter) which is carried out in the Prilling Tower.

The molten urea is pumped to the upper portion of the tower that is 80 m high and 16 m in diameter. A rain of molten urea is obtained, by means of a rotating basket with about 6000 small perforations, the droplets of which first solidify and then cool in the free fall thereof, while air is made to circulate in the opposite direction by means of large fans located in the upper portion of the tower.

In this way the final product is obtained, at a temperature of 40 - 50 °C, which is transported by means of elevators and belts to the storage silos.

### DESCRIPTION OF THE INVENTION

The method for producing organic urea that the present invention proposes is configured, in contrast, as a notable novelty within the field of application thereof, contributing advantages and characteristics that distinguish and improve it with respect to the known method described, which are conveniently contained in the final claims that accompany the present specification.

It is worth mentioning, first of all, that the preparation of urea at an industrial level, both from a hydrocarbon and a biomass is made from the mixture and reaction between liquid ammonia (NH₃) and gaseous carbon dioxide (CO₂). The reaction is verified in two steps (shared by both raw materials):
In the first step, the mixed components form ammonium carbamate.
In the second step, the ammonium carbamate is dehydrated in order to form urea.

The reaction speeds are different. The first step is much faster than the second one, with which the intermediate carbamate accumulates. Furthermore, the first reaction is not completely verified, for which reason ammonia and dioxide also remain free. In addition to this, it must be mentioned that the carbamate is a highly corrosive product, for which reason the portion of carbamate that was not converted to urea is degraded into the original reagents thereof, and is then formed again.

Furthermore, the first reaction is exothermic and the second is endothermic.

An important problem in this process is that in the second step of the reaction, a product called biuret is formed, which results from the union of two urea molecules while losing an ammonia molecule. This product is undesirable due to it being toxic. For this reason it must be eliminated.

Regarding the raw materials, however, it is important to take into account the differences. The raw materials involved in the production of biomass-based organic urea are:
1. the "Organic Ammonia" (NH3) of animal
   - origin, such as excrement (slurry) from pigs, birds, cattle, sheep, the blood from said animals, the hides or organic components thereof such as the bowels, or of
   - plant origin such as Mediterranean pine material, tree and bush leaves, vegetables, fruits or other components derived from forests such as bark or pine nuts.
2. the carbon dioxide (CO2) obtained, for example, from the exhaust gases from the boilers of vapor generators of biogas plants or those produced by heat recovery from plant biomasses,
   in order to form the intermediate compound ammonium carbamate, which is the one that will produce the urea, according to the following diagram:

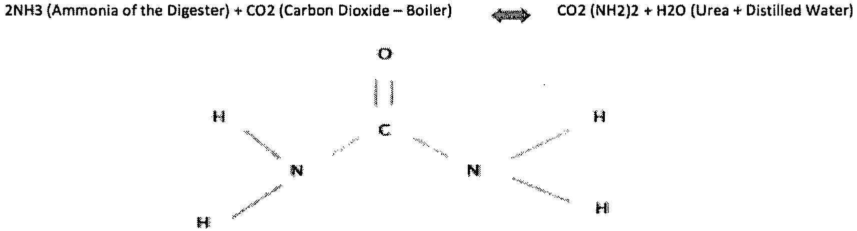

In contrast, the raw materials involved in the production of hydrocarbon-based urea are: the "Ammonia" (NH₃) by cracking the "reformed gas" (cracking of the CH₄molecules) (Hydrocarbons) and the carbon dioxide (CO₂) from natural gas, by means of the reaction known as reforming. Combined, these two elements produce urea.

After establishing the described premises, the method for producing biomass-based organic urea, object of the present invention comprises the following steps:
- Methanization step: The first thing is to load in a biomass digester a compound formed by: "pig slurry, pig blood, chicken slurry, chicken blood, pine needles, pine ashes and water" or any of the other previously described plant/animal/human biomasses. This mixture is heated externally in order to accelerate and produce a chemical reaction that enables methane gas, air, gaseous ammonia and water vapor and a pH regulator to be obtained.
- Catalyzing step: The mass of gases obtained in the previous step is condensed by cooling and the gaseous ammonia is separated to be stored at a pressure of 13 atmospheres. The remaining gaseous ammonia is recirculated to the synthesis loop.
- Carbamate forming step: The urea synthesis reaction is performed at high pressures (200 bar) and the optimal temperature level (190 °C) in a reactor constructed from special stainless steel.

The exhaust gases from the boiler of the methane turbine + plant fuel are introduced into said reactor and it is mixed with the gaseous ammonia.

The ammonium carbamate is produced between the CO₂ and NH₃ creating an exothermic reaction.

The CO₂ and NH₃ components react quickly and exothermically in a first step in order to form the carbamate which is later dehydrated to urea + water.
- Carbamate decomposition step: Not all of the ammonium carbamate decomposes into urea + water. The fraction that decomposes to form urea in relation to the total amount is called: "Conversion"

The Conversion is to the order of 70%. In other words, from every 100 kg of carbamate that is formed, only 70 kg go on to form urea. The rest must be permanently and continuously recycled in order to reach a complete conversion.

Given that the carbamate is formed much faster than the urea, and due to being a highly corrosive product, it is hard to handle. For this reason it must be degraded back to NH₃ and CO₂ in order to then form it again.

Said degradation is preferably performed, since it is cheaper, by stripping the ammonia, moving the reaction towards the products that form it. As the partial pressure of the reagent is lowered, the system reacts towards the equilibrium by degrading the carbamate. This option has the advantage of being able to make the "synthesis pressure", which reduces the recompression cost.
- Urea synthesis step: The carbamate is dehydrated to urea by means of the reaction:

   NH₂ - COONH₄(l)..........NH₂ - CO - NH₂(l) - NH₂(l) + H₂0(l) H= +15.5 kJ/mol

This is an endothermic reaction. The kinematics of the reaction increase with the temperature (the temperature generated can be used by the boiler), increasing the NH₃/CO₂ ratio and decreasing the presence of water.

The urea production is achieved in a vertical reactor which operates at 188 - 190 °C and 160 absolute Kgf/cm2, a N/C ratio of 3.6 - 3.8, an approximate residence time of 45 minutes and a degree of conversion (in one step) of 65 to 70%.

One of the options for optimizing the process is carrying out combined operations for the formation of carbamate (exothermic and quick) in the lower portion of said reactor by feeding excess CO₂ and NH₃ and decomposing the carbamate into urea (much slower and endothermic).

This step is fundamental in order to reduce biuret, requiring a urea concentration of 80% to be reached.

Biuret is formed when two urea molecules bond, releasing an ammonia molecule.

2 NH₂ - CO - NH₂.........NH₂ - CO - NH - CO - NH₂ - NH₃

It is a highly toxic substance for plants and inconvenient for the use thereof in products for reducing nitrogen oxide emissions, such as the chemical reactor AUS32 *(AdBlue*®*).* In order to lower the concentration thereof in values less than 0.35%, an excess of ammonia must be reached in the urea synthesis (for this reason combined plant and animal biomasses are preferably used). This can be achieved by means of a vacuum concentrator which is heated (by applying external heat) by using the residual water vapor from the vapor generator. This method is called synthesis urea which, once achieved, is pumped to an evaporation unit. - Evaporation Step: The flow from the concentrator continues to be concentrated in two evaporation steps, the first of which (it is concentrated by up to 95%) is operating at 0.3 absolute kg/cm2 and the second (it is concentrated up to 99.8%) at a very high vacuum, in order to achieve the evaporation of the water without thermally decomposing the urea. A large-scale ejector must be used in order to achieve the required vacuum levels.

In this way molten urea at 132°C with low water content (approx. 0.5%) is obtained.

This flow can be sent to a Prilling or granulation Tower in order to form urea beads.

As indicated in previous sections, urea has multiple applications and uses, for which reason it is an interesting product with great possibilities for the industrial production thereof, although, due to being organic urea from biomasses the possibilities of use and the advantages are much greater.

Due to the high nitrogen content thereof, commercially prepared urea is used in manufacturing agricultural fertilizers, as a stabilizer in carbon-cellulose explosives or as a basic component of synthetically prepared resins. Likewise, it is used in feed for ruminants as a dietary supplement. In dermatology, it is also used as a natural moisturizer. It is present in adhesives, plastics, resins, dyes, pharmaceutical products and finishes for textile products, paper and metals.

Furthermore, the positive socioeconomic effects of this re-engineering in the manufacturing of organic urea in the industry are obvious:
fertilizers are critical to reaching the agricultural production level necessary to feed the worldwide population, which is quickly increasing.

Thus, one of the advantages derived from the method is the optimization of existing effluent treatment and slurry plants in Spain and other parts of the world originally intended as biogas factories or waste treatment for generating electric energy.

Specifically, the method for producing organic urea from biomasses enables multiple actions to be carried out in said factories such as:
- Activating the generation of the ammonia from the same slurry producing farm.
- Incentivizing the generation thereof during the farm - factory biogas transfer.
- Modifying the current digestion reactor in order to optimize the biological processes (described above) in order to obtain: Methane gas (electricity generator) and organic urea from biomasses.
- Modifying the exhaust gas outlet from the vapor boiler, redirecting it to the digester in order to use all the carbon dioxide that is currently released into the atmosphere, using the temperature of the exhaust gases in order to increase the digestion times in the generator and in the successive processes to obtain ammonia, the formation of carbamate, the decomposition thereof, the synthesis of the urea, the formation of the biuret, the concentration and the evaporation (all these processes have been explained above).
- In addition, using a closed circuit of gases that enables the toxic gases to be recovered such as ammoniacal nitrogen that is currently released into the atmosphere, complying with and improving upon that imposed by the current regulation of the EU ETS for the period after 2012.
- Generating a final compost with very low ammonia content to be used as an active compost, which does not damage the environment.
- Using the plant biomass that is generated, minimizing the risk of forest fires and generating workforce and modernization needs for the gathering and transfer thereof to the biogas treatment plants.
- Using animal blood (depending on the weight/size thereof) as a raw material for obtaining ammonia which is currently used mainly as a raw material for flour of animal origin (one of the main causes of the disease known as: "Bovine spongiform encephalopathy or Mad Cow Disease".
- Eliminating the environmental contamination in the effluents from slaughterhouses of all kinds (Sheep, Cattle, Pig, Bird, etc.).
- Using the obtained organic urea from biomasses as a fundamental component in the preparation of the organic chemical reactor AUS32 *(AdBlue*®*),* which is an element that reduces the nitrous oxide emissions produced by the exhaust gases from vehicles, homes, industries, etc.

Ultimately, the method implies a great advantage regarding the contribution of indirect positive effects on the environment that come from the suitable use of plant and animal biomasses; furthermore, the manufacture of organic-based fertilizers that enable agriculture to be intensified on existing land, reducing the need to expand it to other plots of land that could have different natural or social uses.

Furthermore the negative environmental effects are substantially reduced, which tend to be severe when coming from the production of hydrocarbon-based fertilizers. The wastewater is not a problem. Due to having been treated as part of the process it is slightly acidic (depending on the type of plant), and the contents thereof of toxic substances are minimal (concentrations of: ammonia or ammonium compounds, urea, cadmium, arsenic, fluorides and phosphate).

The treatment of the water as an active agent in the manufacturing of the organic urea causes the effluents thereof, suspended total solids, nitrate and organic nitrogen, phosphorus, potassium, and (as a result), they may be within the normal parameters for fertirrigation in BOD (biological oxygen demand) and COD (chemical oxygen demand).

The process of obtaining organic urea prevents the contamination that is currently produced known as "eutrophication of surface water or nitrogen contamination of groundwater".

### DESCRIPTION OF THE DRAWINGS

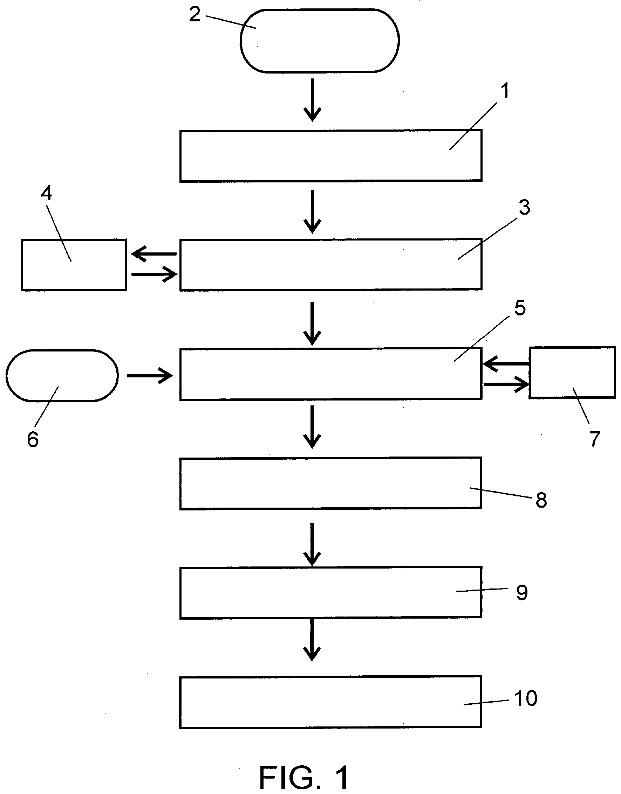

As a complement the present description, and for the purpose of helping to make the characteristics of the invention more readily understandable, the present specification is accompanied by a set of drawings, constituting an integral part of the same, which by way of illustration and not limitation represents the following:
Figure number 1 (only figure). - Shows, by means of a flowchart, a diagram of the steps of the method for producing organic urea, object of the invention.

### PREFERRED EMBODIMENT OF THE INVENTION

In light of the first and only figure, and according to the numbering adopted therein, it can be seen how the method for producing organic urea that the invention proposes comprises the following steps:
- Methanization step (1): in which animal and plant biomass (2) is mixed in a biomass "digester", which is made up of: "pig slurry, pig blood, chicken slurry, chicken blood, pine needles, pine ashes and water" which is externally heated in order to obtain: methane gas, air, gaseous ammonia and water vapor and a pH regulator.
- Catalyzing step: (3), where the mass of gases obtained in the previous step is condensed by cooling and the gaseous ammonia is separated to be stored at a pressure of 13 atmospheres. The remaining gaseous ammonia (4) is recirculated to the synthesis loop.
- Carbamate forming step (5) where the urea synthesis reaction is performed at high pressures (200 bar) and the optimal temperature level (190 °C) in a reactor constructed from special stainless steel.

The exhaust gases (6) from the boiler of the methane turbine + plant fuel are introduced into said reactor and it is mixed with the gaseous ammonia.

The ammonium carbamate is produced between the CO₂ and NH₃ producing an exothermic reaction.

The CO₂ and NH₃ components react quickly and exothermically, in a first step, in order to form the carbamate which is later dehydrated to urea + water.
- Carbamate decomposition step (7): Since not all the ammonium carbamate decomposes into urea + water. The fraction that is not decomposed to form urea is permanently and continuously recycled in order to reach total conversion, being degraded back to NH₃ and CO₂ in order to then form it again.
- Urea synthesis step (8): where the carbamate is dehydrated to urea by means of the reaction:

   NH₂ - COONH₄(l)..........NH₂ - CO - NH₂(l) - NH₂(l) + H₂0(l) H= +15.5 kJ/mol

This is an endothermic reaction. The kinematics of the reaction increase with the temperature (the temperature generated can be used by the boiler), increasing the NH₃/CO₂ ratio and decreasing the presence of water.

Urea production is achieved in a vertical reactor which operates at 188 - 190 °C and 160 absolute Kgf/cm2, a N/C ratio of 3.6 - 3.8, an approximate residence time of 45 minutes and a degree of conversion (in one step) of 65 to 70%.

One of the options for optimizing the process is carrying out combined operations for the formation of carbamate (exothermic and quick) in the lower portion of said reactor by feeding excess CO₂ and NH₃ and decomposing the carbamate into urea (much slower and endothermic).

This step is fundamental in order to reduce biuret, requiring a urea concentration of 80% to be reached. - Evaporation Step (9): The flow from the concentrator continues to be concentrated in two evaporation steps, the first of which (it is concentrated by up to 95%) is operating at 0.3 absolute kg/cm2 and the second (it is concentrated up to 99.8%) at a very high vacuum, in order to achieve the evaporation of the water without thermally decomposing the urea. A large-scale ejector must be used in order to achieve the required vacuum levels.

In this way molten urea at 132°C with low water content (approx. 0.5%) is obtained.

This urea obtained with the method of the invention has been manufactured using biomass, as well as the chemical reactor AUS32 *(AdBlue*®*),* comprises the urea manufactured by the invention.

Optionally, a granulation step (10) is envisaged where the flow can be sent to a Prilling or Granulation Tower, for the formation of urea beads.

Having sufficiently described the nature of the present invention, as well as the ways of implementing it, it is not considered necessary to extend its explanation for any expert in the state of the art to understand its scope and the advantages which derive from it, specifying that, within its essence, it can be carried out in other embodiments that differ in detail from the one provided by way of example, and which are also covered by the requested protection, provided that they do not alter, change or modify its fundamental principle.

## Claims

1. A method for producing organic urea that, made by mixing and reacting preferably gaseous ammonia (NH3) and preferably gaseous carbon dioxide (CO2) in, at least, two steps (5, 8), one where the mixed components form ammonium carbamate, and another where said ammonium carbamate is dehydrated in order to form urea, and preferably envisaging a carbamate forming step (5), a step for degrading carbamate to CO2 and NH3 (7), a urea synthesis step (8) and a water evaporation step (9), is **characterized in that** it is carried out using biomass as a raw material and, in addition to the steps of carbamate formation (5), carbamate degradation (7), urea synthesis (8) and the evaporation thereof (9), it comprises the following prior steps:
- Methanization step (1), where in a biomass "digester" (2) biomass such as pig slurry, pig blood, chicken slurry, chicken blood, pine needles (it acts as a pH regulator), pine ashes (it acts as a pH regulator) and water, and it is externally heated until obtaining: methane gas, air, gaseous ammonia and water vapor; and
- Catalysis step: (3), where the mass of gases obtained in the previous step is condensed by cooling and the gaseous ammonia is separated to be stored at a pressure of 13 atmospheres.

2. The method for producing organic urea according to claim 1, **characterized in that** the ammonia used is organic ammonia from biomasses (2) of animal and plant origin.

3. The method for producing organic urea according to claim 1 or 2, **characterized in that** the carbon dioxide used is carbon dioxide recycled from the exhaust gases (6) from combined-cycle biogas boilers (gas - solids) with plant biomass.

4. The method for producing organic urea according to claim 3, **characterized in that** the carbon dioxide used is that obtained from the exhaust gases from vapor generators of biogas plants and/or the gases produced by heat recovery from plant biomasses.

5. The method for producing organic urea according to claim 1, **characterized in that** in the catalysis step (3) the remaining gaseous ammonia (4) is recirculated.

6. The method for producing organic urea according to claim 1 or 5, **characterized in that** in the carbamate degradation step (7), the ammonium carbamate that is not decomposed into urea and water and, therefore, does not form part of the conversion, is permanently and continuously recycled, being degraded back to NH3 and CO2 in order to then form it again.

7. The method for producing organic urea according to claim 1 or 5 to 6, **characterized in that** in the urea synthesis step (8) the production is produced in a vertical reactor which operates at 188 - 190 °C and 160 absolute Kgf/cm2, a N/C ratio of 3.6 - 3.8, an approximate residence time of 45 minutes and a degree of conversion (in one step) of 65 to 70%.

8. The method for producing organic urea according to claim 7, **characterized in that** in order to optimize the process, combined operations are carried out for the exothermic and quick formation of carbamate in the lower portion of said reactor by feeding excess CO2 and NH3 and decomposing the carbamate into urea, which is much slower and endothermic.

9. The method for producing organic urea according to any of claims 1 or 5 to 8, **characterized in that** in order to lower the biuret concentration in values less than 0.35% and reach an excess of ammonia in the urea synthesis (8) a vacuum concentrator is used, which is heated by using the residual water vapor from the vapor generator in the biogas plant.

10. A method of manufacturing AUS 32 **characterized in that** it comprises urea manufactured using biomass by means of the method of claim 1.
